# FASCICULE DE BREVET EUROPEEN

(11) **EP 4 021 526 B1**
(45) Date de publication et mention de la délivrance du brevet: **09.10.2024**
(21) Numéro de dépôt: 20775699.0
(22) Date de dépôt: 31.08.2020
(51) Int. Cl.: A61M 1/00, A61B 17/00, A61B 17/02, A61B 17/32

(54) **DISPOSITIF CHIRURGICAL COMPORTANT UNE TIGE PRÉSENTANT À SON EXTRÉMITÉ DISTALE UN MANCHON DÉFORMABLE ENTOURANT LADITE TIGE**
CHIRURGISCHE VORRICHTUNG MIT EINEM STAB MIT EINER VERFORMBAREN HÜLSE AN IHREM DISTALEN ENDE, DIE DIESEN STAB UMGIBT
SURGICAL DEVICE COMPRISING A ROD HAVING A DEFORMABLE SLEEVE AT ITS DISTAL END SURROUNDING SAID ROD

(30) Priorité: 30.08.2019 FR 1909554
(43) Date de publication de la demande: 06.07.2022
(73) Titulaire: Institut Hospitalo-Universitaire de Chirurgie Mini-Invasive Guidée par l'Image, 67091 Strasbourg (FR); Institute de Recherche Contre les Cancers de l'appareil Digestif IRCAD, 67091 Strasbourg Cedex (FR)
(72) Inventeur: LEROY, Joël, 62172 Bouvigny-Boyeffles (FR); ALZAGA, Amilcar, Mexico D.F., 03710 (MX)
(74) Mandataire: IP Trust
(86) Numéro de dépôt international: PCT/FR2020/051517
(87) Numéro de publication internationale: WO 2021/038177

(56) Documents cités:
- US-A- 4 324 262
- US-A- 5 171 223
- US-A1- 2014 005 712

## Description

### Domaine de l'invention

La présente invention concerne de manière générale des dispositifs médicaux et plus particulièrement le domaine des dispositifs chirurgicaux destinés à être utilisés dans des procédures chirurgicales mini-invasives ou ouvertes pour la manipulation d'instruments médicaux dans des cavités d'un corps vivant notamment dans le domaine de la cœlioscopie ou de la laparoscopie, comme des crochets pour implanter des fils de suture, des pinces de toute nature pour par exemple découper, prélever, etc. un organe ou une portion d'organe, des électrodes pour par exemple inciser, couper, suturer, coaguler, pour aspirer des fluides et des débris semi-solides, aspirer des fluides en présence de débris volumineux sans se boucher, d'irriguer le champ opératoire, de disséquer et de rétracter des tissus sans les abimer.

De tels dispositifs chirurgicaux sont généralement constitués d'un tube guide comportant une percée traversante de forme cylindrique sensiblement de révolution débouchant respectivement à ses extrémités proximale et distale, d'une tige de commande cylindrique généralement de révolution d'un diamètre extérieur inférieur au diamètre de la percée et montée coulissante dans le tube guide, et de moyens pour accrocher un instrument médical de toute nature, ces moyens d'accrochage étant montés en coopération avec l'extrémité distale de la tige de commande.

Le diamètre extérieur de la tige de commande est déterminé de façon qu'il soit inférieur au diamètre de la percée traversante pour qu'il existe un espace annulaire entre la tige de commande et la paroi de la percée traversante, de façon qu'il soit possible d'insuffler un gaz sous une certaine pression dans le but de dilater la paroi de la cavité dans laquelle doit être utilisé l'instrument, et donc de faciliter le travail du praticien en augmentant l'espace autour du point sur lequel il doit intervenir médicalement.

Le tube peut être de type rigide, pour les applications de coelioscopie et de laparoscopie, ou flexible, pour des applications endoscopiques.

Un endoscope comprend une tige allongée rigide ou un cordon flexible entourée par une gaine étanche. La tige rigide ou le cordon flexible est traversé de manière étanche dans le sens longitudinal par un canal opérateur ouvert à l'extrémité distale et débouchant à l'extrémité proximale dans le corps d'une poignée.

Le canal opérateur est utilisé pour le passage des instruments de travail sur toute la longueur de l'endoscope jusqu'à la zone d'intervention. La gaine qui entoure de manière étanche le canal opérateur contient dans son volume intérieur les dispositifs nécessaires à l'utilisation de l'endoscope, notamment un instrument optique, un dispositif d'éclairage, en forme par exemple de faisceau de fibres optiques classique, ou d'autres dispositifs tels que des dispositifs électroniques ou des instruments d'endothérapie ou de microchirurgie.

Le canal opérateur est aussi utilisé pour la circulation d'un fluide. A cet effet, il est prévu un conduit de sortie latéral, auquel peuvent être raccordés des tuyaux d'écoulement de prolongement.

En règle générale, l'extrémité proximale du canal opérateur peut être fermée du côté de la poignée par une valve et le conduit de sortie peut être ouvert par une autre valve, afin qu'un liquide de nettoyage, par exemple, puisse être injecté à travers la partie distale du canal opérateur vers la zone d'intervention ou aspiré à partir de celle-ci. Le tuyau d'écoulement peut aussi, par exemple, être utilisé pour l'aspiration ou l'admission de gaz.

### État de la technique

On connaît en particulier dans l'état de la technique une solution décrite par la demande de brevet US2014/005712 décrivant différentes variantes de dispositifs pour l'utilisation dans l'ensemble du corps, notamment pour débarrasser le système vasculaire d'obstructions, par un tête en forme d'entonnoir réalisé en un matériau maillé permettant de fixer des débris dans le système vasculaire, par exemple un un caillot sanguin, une plaque, du cholestérol, un thrombus, des corps étrangers naturels (c'est-à-dire une partie du corps logée dans la lumière), un corps étranger non naturel tel qu'une partie d'un dispositif médical ou une autre substance non naturelle logée dans la lumière. Le dispositif d'entonnoir décrit dans ce brevet antérieur comprend une tige flexible, un entonnoir présentant une ouverture distale et une partie proximale reliée à la tige et une cavité entre celles-ci de telle sorte que lorsque l'entonnoir est déployé, l'entonnoir se rétrécit dans une direction proximale vers la tige.

L'entonnoir en treillis est souple et la paroi intérieure en treillis et la paroi extérieure en treillis peuvent coulisser l'une par rapport à l'autre de telle sorte que le retrait du dispositif provoque un mouvement de la paroi intérieure en treillis tandis qu'une partie de la paroi extérieure en treillis reste fixe lorsqu'elle est positionnée contre la paroi.

La déformation de cet entonnoir est assurée par un mouvement de translation de la tige flexible.

On connaît aussi le brevet US4324262 concernant un exemple de réalisation d'une sonde adaptée a l'introduction dans une cavité du corps, tel qu'un tube bronchial ou le poumon, à des fins de biopsie, d'introduction et/ou de prélèvement d'un fluide. A la suite de l'introduction de la sonde dans un tube bronchial, le tube interne est allongé pour ouvrir et passer au travers du repli, roulant ainsi la paroi interne autour de la partie repliée et en renversant les parois internes de la chambre primitive.

Le brevet US5171223 décrit un conduit de drainage et d'instrument résistant à la luxation pour l'arthroscopie comprend un cylindre creux et un manchon déplaçables longitudinalement sur celui-ci qui sont interconnectés à une extrémité, tandis que le manchon, dans la zone de son extrémité de connexion, est construit de telle manière que, lorsque le manchon est déplacé vers le plan de connexion, une zone terminale du manchon est dilatée vers l'extérieur à la manière d'un panier sur la circonférence du manchon, l'extrémité de manchon expansée en forme de panier étant pourvu d'une pluralité de perforations disposées de manière à être réparties sur la circonférence et en agencement côte à côte. Le manchon est arrêté sur le cylindre creux à l'état expansé de la section terminale du manchon.

### Inconvénients de l'art antérieur

Les solutions de l'art antérieur ne sont pas totalement satisfaisantes.

La solution décrite dans le brevet US2014/005712 propose un manchon déployable par un guide longitudinal, pour former un sac de collecte pour récupérer les éléments qui pourraient obstruer la canule. La récupération préalable des débris empêche la canule de se boucher, mais une étape supplémentaire doit être effectuée lors de l'intervention chirurgicale : l'évacuation des déchets récupérés dans le sac de collecte. Cela peut engendrer un allongement du temps de l'intervention.

Le fonctionnement des solutions de l'art antérieur ne permettent pas d'appréhender de manière efficace un débris, car le déplacement en translation peut conduire à repousser le débris dans le conduit vasculaire, et l'entraîner éventuellement dans une bifurcation vers un conduit secondaire où il sera plus difficile à appréhender.

### Solution apportée par l'invention

Afin de remédier à ces inconvénients, l'invention concerne selon son acception la plus générale un dispositif chirurgical conforme à la revendication 1.

Avantageusement ledit élément de commande est formé par un tube entourant ladite tige ou dudit cordon flexible.

Selon une première variante, ledit manchon déformable est formé par un film poreux.

Selon une deuxième variante, ledit manchon déformable est constitué par un treillis de fils.

Selon une troisième variante, ledit manchon déformable est constitué par un treillis de fils métalliques en acier.

Selon une quatrième variante, ledit manchon déformable est constitué par un treillis de fils d'acier inoxydable d'une section comprise entre 150 et 300 micromètres.

De préférence, ledit manchon déformable forme un filtre présentant des lumières de section inférieure à 2 millimètres.

Avantageusement, le dispositif comporte une poignée comportant un bouton de commande mobile selon une course linéaire, pour déplacer l'extrémité proximale du manchon entre une position de repos et une position repliée, et une course angulaire lorsque ledit bouton de commande a atteint ladite position repliée.

### Description détaillée de l'invention

La présente invention sera mieux comprise à la lecture de la description qui suit, se référant à un exemple non limitatif de réalisation de l'invention illustré par les figures annexées où :

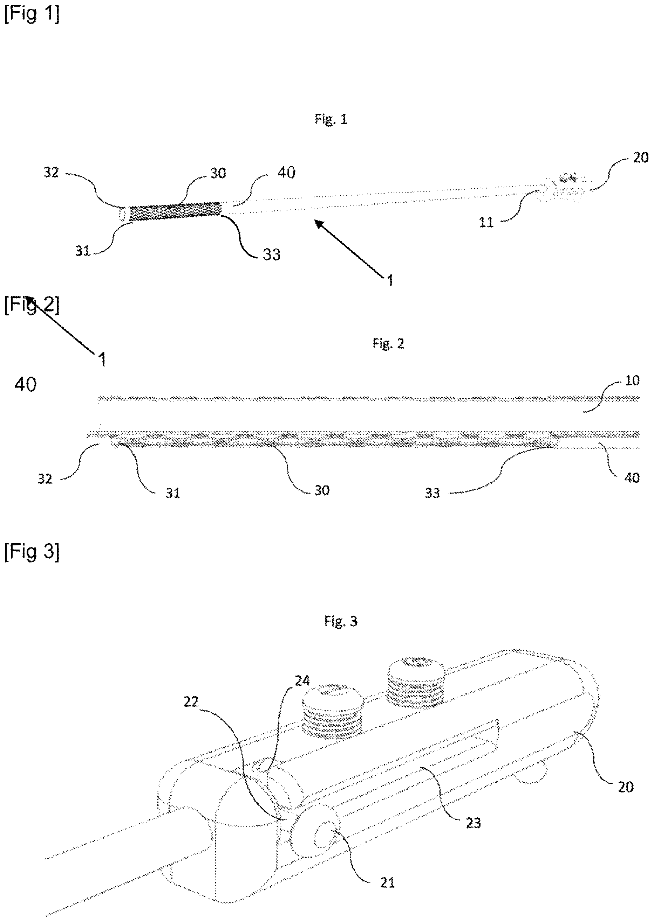

[Fig 1]La figure 1 représente une vue en perspective d'un dispositif selon l'invention
[Fig 2]La figure 2 représente une vue en écorché partiel du guide du dispositif selon l'invention
[Fig 3]La figure 3 représente une vue en perspective de la poignée du dispositif selon l'invention
[Fig 4]La figure 4 représente une vue en perspective ¾ avant de l'extrémité distale en position ouverte
[Fig 5]La figure 5 représente une vue en en coupe selon un plan longitudinal de l'extrémité distale en position ouverte
[Fig 6]La figure 6 représente une vue en perspective ¾ avant de l'extrémité distale en position fermée
[Fig 7]La figure 7 représente une vue en en coupe selon un plan longitudinal de l'extrémité distale en position fermée.

### Description matérielle du dispositif

Par convention on désignera dans le présent brevet par « distales » les parties les plus éloignées de la poignée, et par « proximales » celles qui sont les plus proche de l'opérateur.

Le dispositif chirurgical comprend de manière connue une tige tubulaire (1) dont l'extrémité proximale (11) est engagée dans une poignée (20) comportant les vannes de commande des conduits d'aspiration et d'injection, les raccords avec des équipements complémentaires et les boutons de commande.

La tige tubulaire (1) selon l'invention est constituée d'un élément tubulaire intérieur creux (10) entouré par une gaine (40) coulissant autour de l'élément tubulaire creux (10), avec deux degrés de liberté, à savoir :
a) une liberté de mouvement en rotation relative de l'élément tubulaire intérieur creux (10) par rapport à la gaine (40) et
b) une liberté de mouvement en translation relative de l'élément tubulaire intérieur creux (10) par rapport à la gaine (40).

La tige tubulaire (1) formée par l'élément tubulaire intérieur creux (10) et la gaine (40) présente par ailleurs une flexibilité permettant de déformer la tige tubulaire (1) pour suivre la conformation anatomique du vaisseau à traiter.

L'extrémité distale de la tige (1) est entourée par un manchon déformable (30) de forme tubulaire, entourant le segment distal de l'élément tubulaire (10). Ce manchon (30) est de préférence formé par un ensemble maillé de fils et s'étend entre l'extrémité distale de l'élément tubulaire (10) et l'extrémité distale la gaine (40). Ces deux extrémités du manchon (30) sont fixées respectivement sur l'extrémité de l'élément tubulaire (10) et l'extrémité de la gaine (40) afin de permettre d'exercer une déformation par rétractation, par un déplacement relatif en translation de l'élément tubulaire intérieur creux (10) par rapport à la gaine (40), et en torsion, par un déplacement relatif en rotation de l'élément tubulaire intérieur creux (10) par rapport à la gaine (40).

Ce déplacement relatif est commandé par une poignée (20) comportant des moyens d'actionnement des extrémités proximales de l'élément tubulaire intérieur creux (10) par rapport à la gaine (40), en translation et en rotation.

La poignée (20) présente par ailleurs :
- un canal d'aspiration relié à un dispositif d'aspiration
- un canal d'irrigation relié à un dispositif d'irrigation Les deux canaux se rejoignent en un seul au niveau de l'extrémité proximale des deux tubes concentriques de telle sorte que succion et irrigation se font par le même orifice à l'extrémité distale de la canule ;

- Un bouton pour actionner l'aspiration
- Un bouton pour actionner l'irrigation
- Un bouton pour actionner le déploiement du dispositif anti-obstruction qui peut être tout type de bouton comme un bouton-poussoir, une molette, un bouton coulissant, une gâchette...

L'élément tubulaire (10) est fixé par son extrémité proximale à la poignée (20) et permet l'administration de fluides d'irrigation et l'élimination des fluides corporels et des débris.

Le bord annulaire distal (31) du manchon (30) est solidarisé avec l'extrémité distale de l'élément tubulaire (10) par une bague de scellage (32) venant lier à la fois en translation longitudinale et en rotation l'extrémité distale (31) du manchon (30) avec l'extrémité distale de l'élément tubulaire (10). L'extrémité proximale (33) du manchon (30) est solidaire de la gaine (40).

L'extrémité proximale (33) du manchon (30) est solidaire, en translation et en rotation, d'une gaine (40) présentant une rigidité lui permettant de se déformer autant que l'élément tubulaire et suffisante pour transmettre un effort de déplacement longitudinal et/ou de rotation axial exercé au niveau de la poignée, jusqu'au manchon (30).

Cette gaine (40) est coaxiale avec l'élément tubulaire intérieur (10). Elle est par exemple constituée d'une gaine en un profilé métallique, par exemple en aluminium. Il s'étend depuis l'extrémité proximale (33) du manchon (30) jusqu'à la poignée (20). Cette poignée (20) comprend un mécanisme d'actionnement du manchon (30) formé par un bouton (21) de commande prolongeant un levier de transmission (22) glissant dans une grille présentant un premier segment (23) longitudinal, débouchant à son extrémité distale sur un segment arqué (24).

L'utilisateur peut déplacer le bouton (21) avec son doigt en le faisant d'abord glisser le levier (22) en direction distale, puis, lorsque le levier arrive en butée contre le fond du segment longitudinal (23), à faire pivoter axial dans la rainure (22) pour exercer un couple de torsion sur la gaine (40).

### Description du manchon

On entendra par « manchon » au sens du présent brevet un élément présentant au repos une forme cylindrique, engendrée par des génératrices parallèles. Le manchon déformable (30) présente au repos et avant déformation une section transversale sensiblement constante, ouvert à ces deux extrémités avec une section transversale de ces ouvertures de forme circulaire, c'est-à-dire une surface dans l'espace constituée de droites parallèles. Le manchon (30) est formé par un entrelacement de fils disposés hélicoïdalement, selon deux séries de fils se croisant selon un angle constant pour former des mailles régulières dans une enveloppe tubulaire de section transversale constante.

Le manchon (30) forme au repos un élément tubulaire dont la section intérieure correspond, au jeu près, à la section extérieure de l'élément tubulaire (10).

Le manchon (30) est formé par un treillis de fils métalliques, par exemple des fils d'acier inoxydable d'une section comprise entre 150 et 300 micromètres formant des mailles avec des lumières d'une section de 2 millimètres ou moins.

### Description fonctionnelle

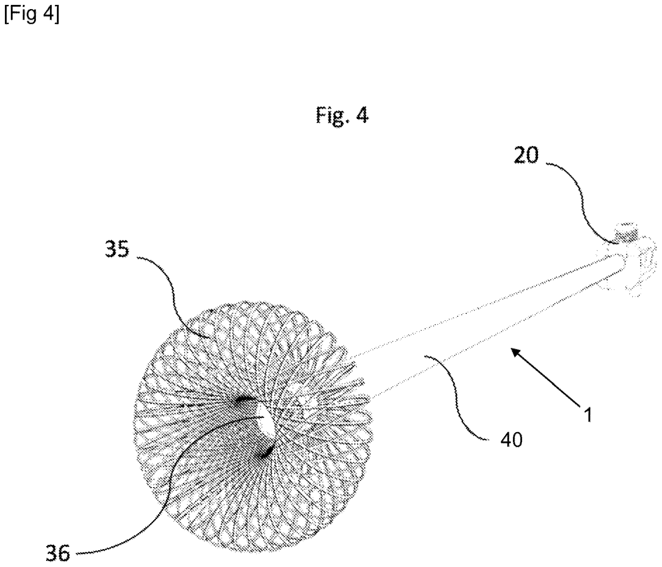

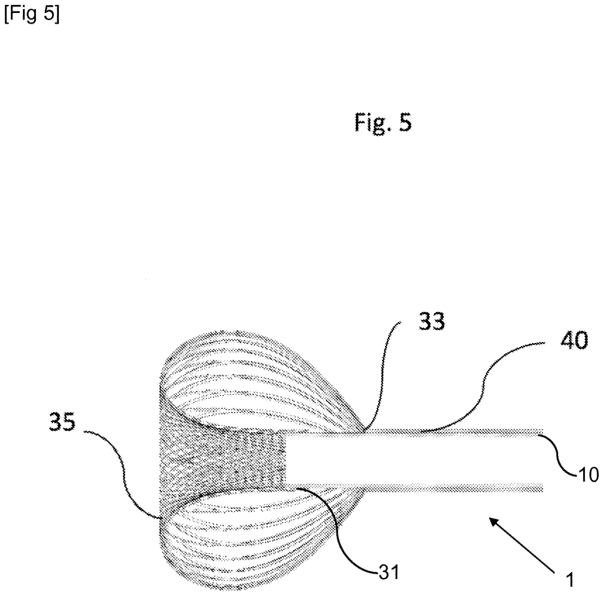

Lorsque l'utilisateur pousse en direction distale la gaine (40), celle-ci repousse l'extrémité arrière (proximale) du manchon (30) qui va être soumis à une contrainte le déformant, en le « rattatinant » sur lui-même, puis en provoquant un épanouissant en forme de tore (35) lorsque l'extrémité proximale (33) du manchon se rapproche de son extrémité distale (31). Les figures 4 et 5 illustrent cette situation qui se produit lorsque le manchon exerce un effort uniquement longitudinal, sans rotation. Le tore (35) qui se forme est ouvert avec un puit central (36), et permet le passage des flux et débris dans le conduit sans aucune obturation.

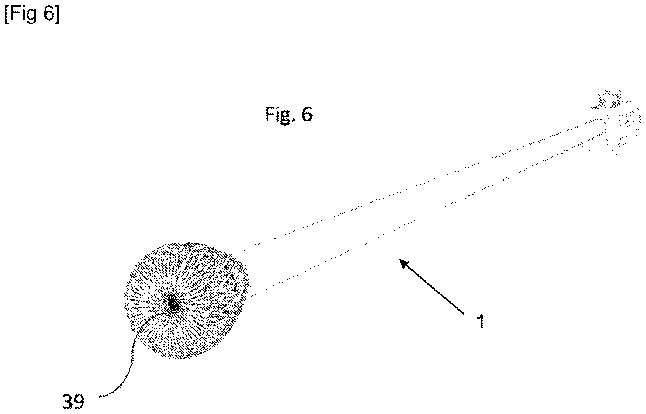

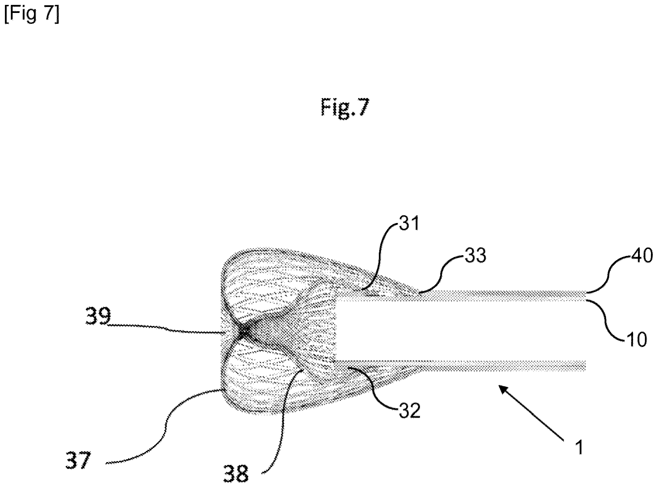

Lorsqu'on provoque un effort angulaire sur la gaine (40), le manchon (30) se tord, ce qui provoque la formation d'un double-tore (37, 38) avec une intersection fermée (39) formant un étranglement obturant la veine d'entrée de la tige (1). Les figures 6 et 7 illustrent cette situation.

Ce dispositif permet de passer facilement d'une canule ouverte à une canule fermée par le manchon (30) grâce au bouton de déploiement (21) présent sur la poignée (20) et d'éviter au chirurgien de ressortir l'aspirateur du patient s'il veut passer d'une canule ouverte à fermée.

Le manchon filtrant (30) peut donc être dans trois états différents :
- Rétractée = autour et le long de l'élément tubulaire (10) pour l'insertion au travers d'un trocart
- Déployée par translation laissant l'orifice de l'extrémité distale ouvert pour l'aspiration de petits débris (position déployée ouverte)
- Déployée par translation/rotation qui permet une clôture de l'orifice de l'extrémité distale pour empêcher l'obstruction du dispositif (position déployée fermée). Dans cette position, le manchon présente une rigidité permettant à l'utilisateur de le mettre en contact avec les tissus sur lesquels il souhaite aspirer du liquidesans que le manchon ne s'écrase sur lui-même.

Un aspirateur irrigateur selon l'invention pourra avoir d'autres usages décrit plus bas tels que la rétraction d'organes et de tissus ainsi que la dissection mousse de tissus ou structures anatomiques. Afin de permettre la dissection mousse, il est indispensable que l'extrémité distale du dispositif possède une rigidité suffisante afin d'exercer un effort suffisant sur les tissus lors de l'appui de l'instrument sur les tissus afin de les disséquer.

Un tel état de rigidification de l'extrémité du dispositif pourra notamment être atteint en appliquant à l'extrémité proximale (33) du manchon (30) une translation dans la direction proximale après que celui-ci soit ait été placé en position déployée par translation/rotation.

Cette translation dans la direction proximale de l'extrémité proximale (33) du manchon (30) pourra être appliquée en appliquant une translation dans la direction proximale à la gaine (40) solidaire de l'extrémité proximale (33) manchon (30).

Cette translation dans la direction proximale de l'extrémité proximale (33) du manchon (30), une fois que celui-ci est dans un état déployé par translation/rotation, a pour effet de « tasser » le manchon augmentant ainsi sa rigidité et d'atteindre un nouvel état.

Dans une version de l'invention, l'organe de commande est adapté afin de permettre la translation en direction proximale après la translation dans la direction distale et la rotation du manchon.

Dans une sous-variante où la commande est manuelle et réalisée par la main d'un opérateur, cette translation en direction proximale du manchon est réalisé au moyen d'une poignée adaptée à la préhension comprenant par exemple une glissière.

Dans une sous-variante où la commande est manuelle et réalisée par la main d'un opérateur, cette translation en direction proximale du manchon est réalisée au moyen d'une poignée adaptée à la préhension comprenant par exemple une glissière, la poignée comprenant en plus un ressort. Ce ressort peut être par exemple comprimé par l'utilisateur lorsque celui-ci déplace une glissière afin de réaliser la première translation, l'énergie du ressort étant libérée, après la rotation, afin de réaliser la seconde translation dans la direction proximale cette fois.

### Utilisation du dispositif

Le dispositif selon l'invention permet de commander depuis la poignée une modification de la configuration d'entrée de la tige pour permettre de laisser passer dans le conduit d'aspiration l'intégralité des flux, ou de filtrer sélectivement les composants en fonction de leur taille et/ou de leur viscosité. Le dispositif selon l'invention permet une commutation facile entre le mode filtration où l'extrémité distale est perméable uniquement aux liquides et le mode aspiration de caillots sanguins, de débris ou de solides.

Le manchon filtrant (30) en position rétractée, est d'une épaisseur telle qu'il permet de pénétrer sans effort et sans risque dans les tailles d'incision classiquement présentes lors d'interventions chirurgicales mini-invasives. Une fois dans la cavité corporelle, le manchon filtrant (30) peut être déployé facilement et rapidement par action d'un bouton dédié présent sur la poignée. Lorsqu'il est déployé, le manchon filtrant (30) recouvre partiellement ou complètement le trou d'aspiration, empêchant ainsi les débris et les solides de gêner le passage des fluides. La nature poreuse du filtre permet une aspiration sans entrave des fluides corporels.

Un inconvénient majeur des dispositifs connus réside dans le fait que les organes et tissus mous (l'intestin grêle, les tissus adipeux, le mésentère, l'omentum) ou les gros caillots peuvent facilement se retrouver aspirés dans la lumière de la canule. En chirurgie mini-invasive, le chirurgien doit alors stopper l'aspiration et utiliser un autre outil pour déboucher l'aspirateur, et s'il n'y parvient pas, il faut alors ressortir l'aspirateur pour le nettoyer à l'extérieur du patient. Il peut être nécessaire de déboucher le dispositif d'aspiration plusieurs fois au cours d'une seule opération chirurgicale avec pour conséquences :
- de perturber le déroulement du travail du chirurgien et du reste du personnel,
- et d'allonger la durée des interventions
- et donc d'augmenter
   o∘ les risques pour le patient
   ∘ la fatigue de l'équipe chirurgicale
   ∘ et le coût total de l'opération.

L'interruption intempestive de la succion est un problème majeur présenté par les aspirateurs-irrigateurs disponibles.

La plupart des solutions existantes présentant un dispositif qui permet d'empêcher la canule de se boucher, ne permettent pas l'aspiration de débris semi-solides.

Il permet également d'apporter des fonctionnalités nouvelles, à savoir l'aspiration de fluides et/ou débris semi-solides, la filtration de débris solides, la dissection de tissus et la rétraction d'organe en toute sécurité. Le manchon filtrant (30) déployable permet de filtrer les débris solides et de pratiquer de la dissection mousse en tout sécurité, tout en conservant la possibilité d'aspirer les fluides et les débris semi-solides lorsque le filtre est en position rétracté.

Les chirurgiens ont pris pour habitude d'utiliser les dispositifs de succion-irrigation dans d'autres buts que d'aspirer et irriguer, et notamment pour disséquer des tissus et déplacer des organes. Les dispositifs d'aspiration connus sont généralement faits d'une canule métallique non protégée à l'extrémité distale. Il existe donc une possibilité de causer des lésions tissulaires lors de l'usage de ces dispositifs dans des utilisations non prévues à l'origine.

La rétraction consiste à déplacer ou écarter de champ de vision opératoire des organes. Par exemple en chirurgie digestive, pour de nombreuses opérations, il est nécessaire d'écarter le foie et de le maintenir ainsi écarté pendant toute la durée de l'intervention afin d'accéder puis d'intervenir sur les organes situés en-dessous. Le but étant de maintenir l'organe hors du champ de vision, l'opération de maintien de l'organe dans cette position écartée n'est pas faite sous le contrôle de la vue. Il est déterminant que l'instrument utilisé soit atraumatique afin de ne pas léser les organes rétractés. Des dispositifs dédiés à la rétraction de tissus compatibles avec les approches endoscopiques et notamment coelioscopiques ont été développés, comme par exemple les rétracteurs en éventail. Cependant leur utilisation requiert le retrait d'un autre instrument chirurgical afin de permettre l'insertion de ces dispositifs dédiés exclusivement à la rétraction de tissus. Le dispositif selon l'invention permet avantageusement de réaliser de la succion ou de l'irrigation puis de rétracter ou déplacer des tissus ou organes sans qu'un échange d'instrument ne soit nécessaire.

Parmi les utilisations collatérales, on compte la rétractation douce de tissus : pendant que le manchon filtrant (30) est déployé, il est suffisamment rigide pour permettre une dissection et une rétraction douces des tissus.
- De déplacer des tissus et des organes en toute sécurité grâce au dispositif anti-obstruction qui se déploie au-delà de l'extrémité distale de l'élément tubulaire intérieur (10)
- D'empêcher le contact entre la pointe de la canule et les tissus lors de la succion grâce au déploiement du dispositif anti-obstruction au-delà de l'extrémité distale de l'élément tubulaire intérieur (10).

Lors des interventions chirurgicales plusieurs méthodes de dissections sont utilisés. L'une d'entre est la dissection mousse : celle-ci est réalisée sans couper ni utiliser d'énergie (électricité, ultra-sons). En chirurgie ouverte, elle est réalisé au doigt : le chirurgien sépare les tissus par application d'une pression et en imprimant des mouvement de translations. C'est une dissection très sure et très couramment utilisée.

En chirurgie endoscopique et notamment coelioscopique, le chirurgien ne peut insérer ses mains dans le champ opératoire. Un instrument est donc nécessaire afin de réaliser cette dissection. Des instruments dédiés à la dissection mousse et spécifique à la coeliscopie ont par exemple été développés. Ceux-ci se compose d'un long manche à l'extrémité duquel est fixé un bulbe composé de gauze ou de ouate dense.

Une autre utilisation collatérale de l'invention est la dissection mousse de tissus et structures anatomiques : pendant que le manchon filtrant (30) est déployé, il est suffisamment rigide pour permettre la dissection mousse de tissus.

Des dispositifs dédiés exclusivement à la dissection mousse compatibles avec les approches endoscopiques et notamment coelioscopiques ont ainsi été développés, cependant leur utilisation requiert le retrait d'un autre instrument chirurgical afin de permettre l'insertion de ces dispositifs dédiés exclusivement à la dissection mousse. Le dispositif selon l'invention permet avantageusement de réaliser de la succion ou de l'irrigation puis de la dissection mousse sans qu'un échange d'instrument ne soit nécessaire.

Par exemple, au cours d'une cholécystectomie coelioscopique, l'invention pourra être utilisé en qu'aspirateur irrigateur au cours de la dissection afin de nettoyer le champ opérateur, d'aspirer l'eau utilisée pour l'irrigation, d'aspirer le sang et potentiellement la bile qui pourrait se répandre durant l'intervention. Cette étape pourra être réalisée sans risque d'occlusion si le manchon est en position déployée.

De plus, l'invention pourra être utilisée pour rétracter le foie afin d'exposer la vésicule sans risque de léser la surface de celui-ci.

Enfin l'invention pourra être utilisée afin de disséquer : par exemple pour séparer sans incision ni recours à une source d'énergie la vésicule de la surface du foie. Avantageusement, cette dissection pourra être faite de manière contrôlée et sûre car après déploiement par translation puis rotation, le manchon est homogène et atraumatique mais suffisamment dense pour permettre la dissection mousse.

Dans certaines variantes de l'invention, l'utilisateur aura la possibilité de faire subir une translation dans la direction proximale au manchon après le déploiement par rotation afin de le tasser et donc d'augmenter la dureté du bulbe formé par le manchon. Il est ainsi possible d'adapter la rigidité de l'extrémité du dispositif à la fois aux préférences de l'utilisateur et aux tissus rencontrés.

### Utilisation en robotique

Un aspirateur-irrigateur selon l'invention aura de l'intérêt dans une utilisation robotique où l'échange d'instruments est plus complexe et plus long que dans une approche conventionelle. Par exemple, en coelisocopie robotique, une incision supplémentaire est parfois pratiquée sur l'abdomen du patient afin d'introduire un apsirateur-irrigateur non robotique.

Dans une variante de l'invention, l'organe de commande est une interface robotique adaptée pour être montée sur un bras de robot ou le bras d'un télé-manipulateur.

L'organe de commande pourra par exemple intégrer plusieurs interfaces permettant l'actionnement robotisé de l'instrument. Ceux-ci pourront comprendre des interfaces permettant la mise en mouvement des différents éléments mobiles de l'invention : mise en translation ou en rotation de la gaine ou encore des interfaces permettant l'actionnement de l'aspiration ou de l'irrigation.

Le dispositif selon l'invention est particulièrement adapté à des interventions chirurgicales robotisées, mettant en oeuvre un robot endoscopique. L'introduction et la progression de la tige, puis l'actionnement en translation puis en torsion est assuré par des actionneurs commandés par un calculateur permettant d'automatiser la récupération d'un caillot ou d'un corps à isoler. Cette séquence de translation de l'élément de commande, puis de rotation axiale, puis de translation additionnelle pour tasser le corps pris dans le manchon peut être commandée par le calculateur, par exemple lorsque l'opérateur déclenche le démarrage de la séquence par une instruction unique.

### Applications chirurgicales

Durant la chirurgie vasculaire ou d'autres spécialités chirurgicales se concentrant sur les organes mous (gynécologie, urologie, chirurgie digestive...), l'une des étapes déterminantes est l'accès à la structure ou à l'organe sur lequel ou laquelle l'intervention doit avoir lieu. Plusieurs techniques de dissection sont typiquement utilisés consécutivement pour séparer les tissus, les diviser ou en retirer. Au cours de ces dissections des saignements peuvent avoir lieu, il est dans ce cas intéressant d'aspirer le sang ou les autres fluides polluant la vision du champ opératoire. Parfois il est nécessaire d'aspirer des caillots ou de petits débris solides. Afin de nettoyer le champ opératoire, il est également utile d'utiliser l'irrigation pour retirer les fluides opaques empêchant une bonne visualisation du champ opératoire. Si un aspirateur selon l'invention est utilisé, avec le manchon déployé par translation et rotation, l'aspiration peut être réalisée sans risque d'obstruction intempestive de l'extrémité de la canule. Il ne sera par exemple pas nécessaire d'avoir recours à des compresses ou quelqu'autre filtre improvisé afin d'éviter l'occlusion de l'extrémité.

Si un caillot ou un débris est rencontré au cours de l'intervention, l'utilisateur peut aisément placer le manchon en position rétractée afin de l'aspirer.

Lorsqu'au cours de l'intervention il sera nécessaire de rétracter un organe afin de le maintenir écarté du champ opératoire, l'utilisateur placera le manchon en position déployée par translation et rotation afin d'avoir l'assurance d'un contact atraumatique avec l'organe rétracté. Il ne sera alors pas nécessaire de retirer l'aspirateur du patient pour introduire un rétracteur dédié. Il est par exemple nécessaire de rétracter le foie lors d'une opération de bypass gastrique : le foie est maintenu sur le côté gauche du patient, en contact avec la paroi abdominale afin d'exposer l'estomac et l'intestin grêle qui sont les structures cibles pour cette procédure.

Lorsque de la dissection mousse sera nécessaire afin de séparer des tissus, l'utilisateur pourra placer le manchon en position déployée par rotation et translation afin de séparer les tissus de manière complètement atraumatique. Ici encore il ne sera donc pas nécessaire de retirer un instrument pour introduire un instrument de dissection mousse dédié. L'aspirateur irrigateur selon l'invention pourra être utilisé afin de réaliser des dissections atraumatiques.

Par exemple, dans le cas de l'ablation de la vésicule biliaire, celle-ci doit être séparée du foie. Cette opération peut-être réalisée au moyen de l'invention. L'artère et le canal cystique menant à la vésicule doivent être complètement séparés et isolés des tissus environnants afin de les identifier avec certitude, les clamper er les sectionner afin de libérer la vésicule. Cette étape de dissection est ici encore une séparation de tissus qui pourra avantageusement être réalisé au moyen de l'invention lorsque le manchon est déployé par translation et rotation.

SI au cours de ces dissections l'utilisateur souhaitait aspirer, irriguer le champ ou rétracter un organe il lui suffirait de modifier le déployement du manchon au moyen de l'organe de commande.

## Revendications

1. - Dispositif chirurgical comportant une tige (1) présentant à son extrémité proximale une poignée (20) et à son extrémité distale un manchon déformable (30) entourant ladite tige (1), et un élément de commande (21, 22, 40) mobile longitudinalement; ladite tige (1) étant constituée par un élément tubulaire intérieur creux (10) entouré par une gaine (40) présentant une rigidité lui permettant de se déformer autant que l'élément tubulaire et suffisante pour transmettre un effort de déplacement longitudinal et/ou de rotation axial exercé au niveau de la poignée (20) jusqu'au manchon (30), ladite gaine (40) coulissant autour de l'élément tubulaire creux (10), avec deux degrés de liberté, à savoir :
o une liberté de mouvement en translation relative de l'élément tubulaire intérieur creux (10) par rapport à la gaine (40)
o une liberté de mouvement en rotation relative de l'élément tubulaire intérieur creux (10) par rapport à la gaine (40) et les deux extrémités dudit manchon (30) étant
fixées respectivement sur l'extrémité de l'élément tubulaire (10) et l'extrémité de ladite gaine (40) afin de permettre d'exercer une déformation par rétractation, par un déplacement relatif en translation de dudit élément tubulaire intérieur creux (10) par rapport à ladite gaine (40), et en torsion, par un déplacement relatif en rotation dudit l'élément tubulaire intérieur creux (10) par rapport à ladite gaine (40) ledit élément de commande (21, 22, 40) étant mobile longitudinalement pour déplacer l'extrémité proximale dudit manchon (30) entre :
∘ une position de repos où ledit manchon déformable (30) présente une forme tubulaire,
∘ et une position active où ledit manchon (30) est déformé par rapprochement de ses extrémités distale et proximale
∘ et en rotation pour exercer un effort angulaire sur ladite gaine (40) pour tordre ledit manchon (30) et provoquer la formation d'un double-tore (37, 38) avec une intersection fermée (39) formant un étranglement obturant la veine d'entrée de la tige (1).

2. - Dispositif chirurgical selon la revendication 1 **caractérisé en ce que** ledit manchon (30) forme au repos un élément tubulaire dont la section intérieure correspond, au jeu près, à la section extérieure de l'élément tubulaire (10).

3. - Dispositif chirurgical selon la revendication 1 **caractérisé en ce que** ledit manchon déformable (30) est formé par un film poreux.

4. - Dispositif chirurgical selon la revendication 1 **caractérisé en ce que** ledit manchon déformable (30) est constitué par un treillis de fils.

5. - Dispositif chirurgical selon la revendication 1 **caractérisé en ce que** ledit manchon déformable (30) est constitué par un treillis de fils métalliques en acier.

6. - Dispositif chirurgical selon la revendication 1 **caractérisé en ce que** ledit manchon déformable (30) est constitué par un treillis de fils d'acier inoxydable d'une section comprise entre 150 et 300 micromètres.

7. - Dispositif chirurgical selon la revendication 1 **caractérisé en ce que** ledit manchon déformable (30) forme un filtre présentant des lumières de section inférieure à 2 millimètres.

8. - Dispositif chirurgical selon la revendication 1 **caractérisé en ce que** ladite poignée (20) comporte un bouton de commande mobile (21, 22) selon une course linéaire, pour déplacer l'extrémité proximale du manchon (30) entre une position de repos et une position repliée, et une course angulaire lorsque ledit bouton de commande a atteint ladite position repliée.

## Patentansprüche

1. Chirurgische Vorrichtung, die einen Stab (1), der an seinem proximalen Ende einen Griff (20) und an seinem distalen Ende eine verformbare Hülse (30) vorweist, die den Stab (1) umgibt, und ein in Längsrichtung bewegbares Steuerelement (21, 22, 40) aufweist; wobei der Stab (1) aus einem hohlen inneren röhrenförmigen Element (10) besteht, das durch eine Hülle (40) umgeben ist, die eine Steifigkeit vorweist, die es ihr ermöglicht, sich genauso stark wie das röhrenförmige Element zu verformen, und zum Übertragen einer Längsverschiebungs- und/oder Axialdrehungskraft ausreichend ist, die an dem Griff (20) bis zu der Hülse (30) ausgeübt wird, wobei die Hülle (40) mit zwei Freiheitsgraden um das hohle röhrenförmige Element (10) gleitet, nämlich:
∘ einer Translationsbewegungsfreiheit relativ zu dem hohlen inneren röhrenförmigen Element (10) in Bezug auf die Hülle (40)
∘ einer Drehungsbewegungsfreiheit relativ zu dem hohlen inneren röhrenförmigen Element (10) in Bezug auf die Hülle (40) und
wobei die zwei Enden der Hülse (30) an dem Ende des röhrenförmigen Elements (10) beziehungsweise an dem Ende der Hülle (40) befestigt sind, um es zu ermöglichen, eine Verformung durch Zurückziehen durch eine relative Verschiebung in Translation des hohlen inneren röhrenförmigen Elements (10) in Bezug auf die Hülle (40) und in Torsion durch eine relative Drehverschiebung des hohlen inneren röhrenförmigen Elements (10) in Bezug auf die Hülle (40) auszuüben; wobei das Steuerelement (21, 22, 40) zum Verschieben des proximalen Endes der Hülse (30) in Längsrichtung bewegbar ist zwischen:
∘ einer Ruheposition, in der die verformbare Hülse (30) eine röhrenförmige Form vorweist,
∘ und einer aktiven Position, in der die Hülse (30) durch Zusammenführen ihres distalen und proximalen Endes verformt wird
∘ und in Drehung zum Ausüben einer Winkelkraft auf die Hülle (40) zum Verbiegen der Hülse (30) und Veranlassen der Ausbildung eines Doppeltorus (37, 38) mit einem geschlossenen Schnittpunkt (39), der eine Verengung ausbildet, die den Eintrittskanal des Stabs (1) verschließt.

2. Chirurgische Vorrichtung nach Anspruch 1,
**dadurch gekennzeichnet, dass** die Hülse (30) in Ruhe ein röhrenförmiges Element ausbildet, dessen Innenquerschnitt bis auf ein Spiel dem Außenquerschnitt des röhrenförmigen Elements (10) entspricht.

3. Chirurgische Vorrichtung nach Anspruch 1,
**dadurch gekennzeichnet, dass** die verformbare Hülse (30) aus einer porösen Folie ausgebildet ist.

4. Chirurgische Vorrichtung nach Anspruch 1,
**dadurch gekennzeichnet, dass** die verformbare Hülse (30) aus einem Drahtgeflecht besteht.

5. Chirurgische Vorrichtung nach Anspruch 1 ,
**dadurch gekennzeichnet, dass** die verformbare Hülse (30) aus einem Geflecht aus metallischen Stahldrähten besteht.

6. Chirurgische Vorrichtung nach Anspruch 1,
**dadurch gekennzeichnet, dass** die verformbare Hülse (30) aus einem Geflecht aus Edelstahldrähten mit einem Querschnitt zwischen 150 und 300 Mikrometern besteht.

7. Chirurgische Vorrichtung nach Anspruch 1,
**dadurch gekennzeichnet, dass** die verformbare Hülse (30) einen Filter ausbildet, der Lufteinlässe mit einem Querschnitt von weniger als 2 Millimetern vorweist.

8. Chirurgische Vorrichtung nach Anspruch 1,
**dadurch gekennzeichnet, dass** der Griff (20) einen Steuerknopf (21, 22) aufweist, der in einem linearen Hub, zum Verschieben des proximalen Endes der Hülse (30) zwischen einer Ruheposition und einer eingezogenen Position, und einem Winkelhub bewegbar ist, wenn der Steuerknopf die eingezogene Position erreicht hat.

## Claims

1. Surgical device comprising a rod (1) having at its proximal end a handle (20) and at its distal end a deformable sleeve (30) surrounding said rod (1), and a longitudinally movable control element (21, 22, 40); said rod (1) being made up of a hollow inner tubular element (10) surrounded by a sheath (40) having a rigidity which allows it to deform as much as the tubular element and which is sufficient for transmitting a longitudinal movement force and/or axial rotation force exerted on the handle (20) to the sleeve (30), said sheath (40) sliding around the hollow tubular element (10), with two degrees of freedom, namely:
∘ a freedom of relative translational movement of the hollow inner tubular element (10) with respect to the sheath (40)
∘ a freedom of relative rotational movement of the hollow inner tubular element (10) with respect to the sheath (40), and
the two ends of said sleeve (30) being fixed respectively to the end of the tubular element (10) and the end of said sheath (40) so as to allow a deformation to be exerted by retraction, by relative translational, and torsional, movement of said hollow inner tubular element (10) with respect to said sheath (40), by relative rotational movement of said hollow inner tubular element (10) with respect to said sheath (40); said control element (21, 22, 40) being longitudinally movable to move the proximal end of said sleeve (30) between:
o∘ a rest position in which said deformable sleeve (30) has a tubular shape,
∘ and an active position in which said sleeve (30) is deformed by bringing its distal and proximal ends together
∘ and in rotation to exert an angular force on said sheath (40) to twist said sleeve (30) and cause the formation of a double torus (37, 38) having a closed intersection (39) forming a constriction closing the inlet vein of the rod (1).

2. Surgical device according to claim 1, **characterized in that,** at rest, the sleeve (30) forms a tubular element of which the inner cross-section corresponds, except for play, to the outer cross-section of the tubular element (10).

3. Surgical device according to claim 1, **characterized in that** said deformable sleeve (30) is formed by a porous film.

4. Surgical device according to claim 1, **characterized in that** said deformable sleeve (30) is made up of a mesh of wires.

5. Surgical device according to claim 1, **characterized in that** said deformable sleeve (30) is made up of a mesh of steel wires.

6. Surgical device according to claim 1, **characterized in that** said deformable sleeve (30) is made up of a mesh of stainless steel wires having a cross-section of between 150 and 300 micrometers.

7. Surgical device according to claim 1, **characterized in that** said deformable sleeve (30) forms a filter which has lumens having a cross-section of less than 2 millimeters.

8. Surgical device according to claim 1, **characterized in that** said handle (20) comprises a control button (21, 22) movable along a linear path, to move the proximal end of the sleeve (30) between a rest position and a folded position, and an angular path when said control button has reached said folded position.
